# EUROPEAN PATENT SPECIFICATION

(11) **EP 4 029 435 B1**
(45) Date of publication and mention of the grant of the patent: **01.05.2024**
(21) Application number: 21151839.4
(22) Date of filing: 15.01.2021
(51) Int. Cl.: A61B 5/022, A61B 5/0225, A61B 5/021

(54) **BLOOD PRESSURE MONITORING DEVICE AND METHOD FOR ADAPTIVE BLOOD PRESSURE MONITORING**
BLUTDRUCKÜBERWACHUNGSVORRICHTUNG UND VERFAHREN ZUR ADAPTIVEN BLUTDRUCKÜBERWACHUNG
DISPOSITIF DE SURVEILLANCE DE LA PRESSION SANGUINE ET PROCÉDÉ DE SURVEILLANCE ADAPTATIVE DE LA PRESSION SANGUINE

(43) Date of publication of application: 20.07.2022
(73) Proprietor: Infineon Technologies AG, 85579 Neubiberg (DE)
(72) Inventor: Fusco, Alessandra, 80799 München (DE); Kollias, Athanasios, 81737 München (DE); Poenaru, Ilie, 85653 Goeggenhofen/Aying (DE); Stadler, Michael, 81667 München (DE); Zotes, Mario Alejandro, 81669 München (DE)
(74) Representative: Hersina, Günter

(56) References cited:
- US-A- 4 846 189
- US-A1- 2014 012 147
- US-A1- 2014 296 757
- DING XIAORONG ET AL: "A flexible tonoarteriography-based body sensor network for cuffless measurement of arterial blood pressure", 2015 IEEE 12TH INTERNATIONAL CONFERENCE ON WEARABLE AND IMPLANTABLE BODY SENSOR NETWORKS (BSN), IEEE, 9 June 2015 (2015-06-09), pages 1-4, XP032795078, DOI: 10.1109/BSN.2015.7299405 [retrieved on 2015-10-15]

## Description

### Technical Field

Embodiments according to the present application related to a blood pressure monitoring device and a method for adaptive blood pressure monitoring.

### Background

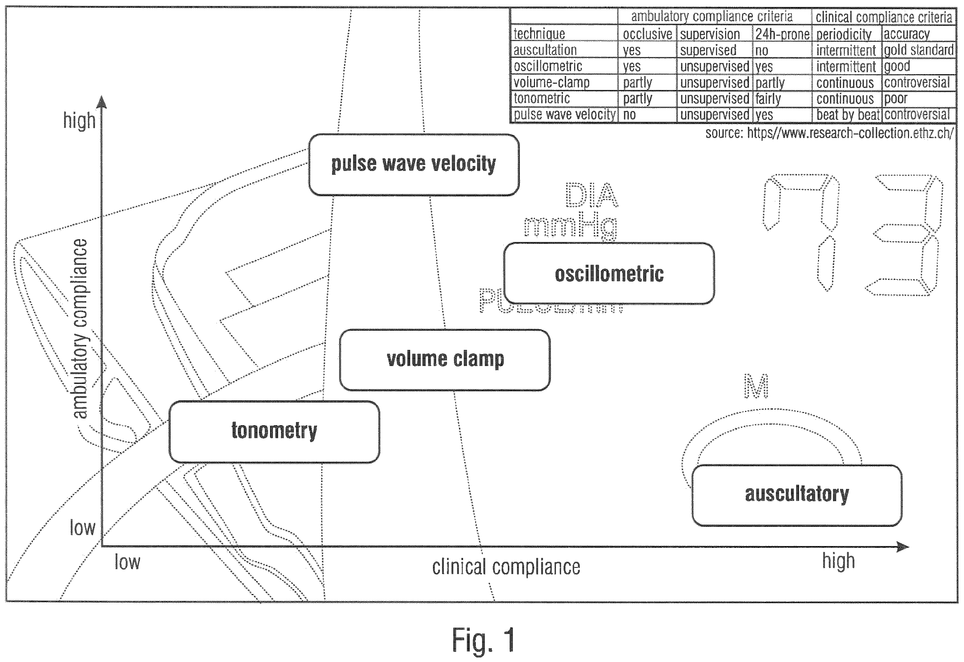

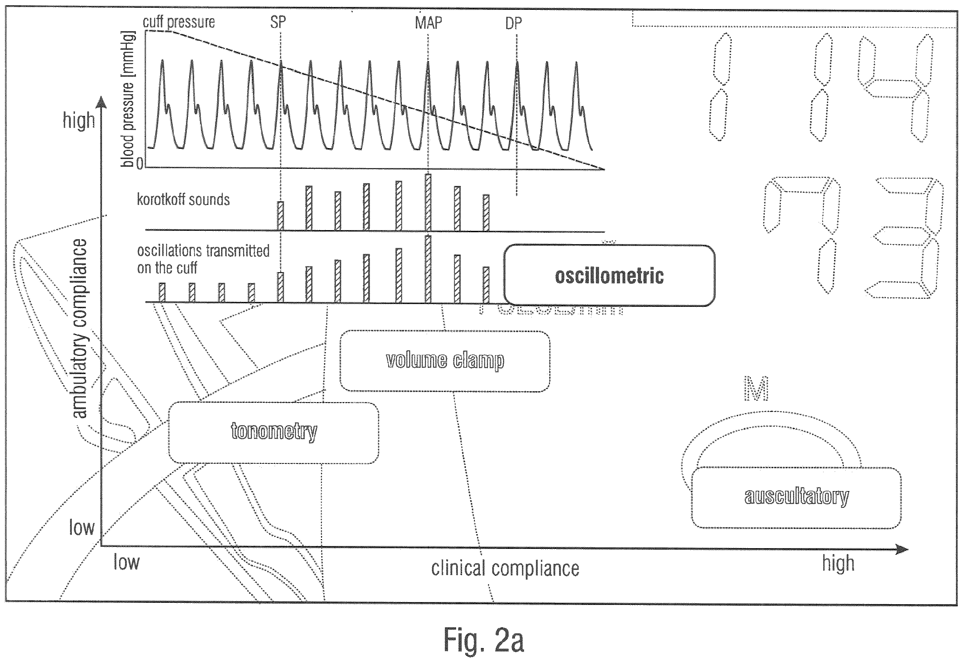

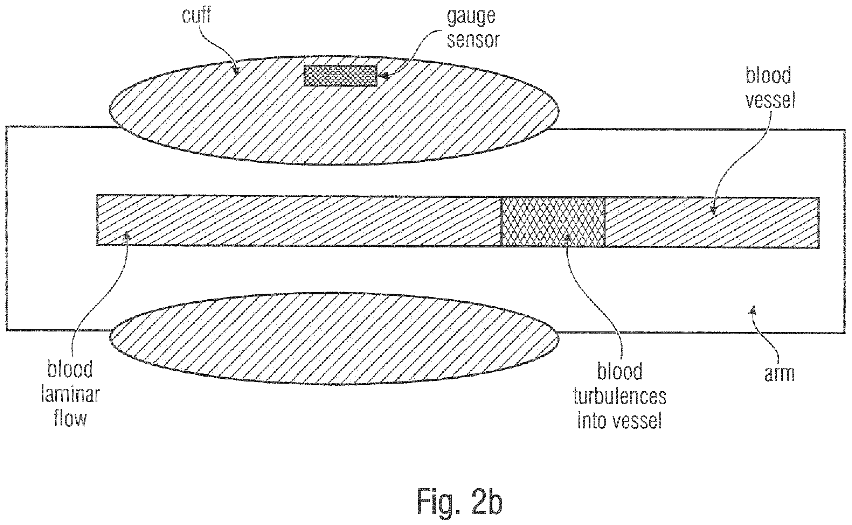

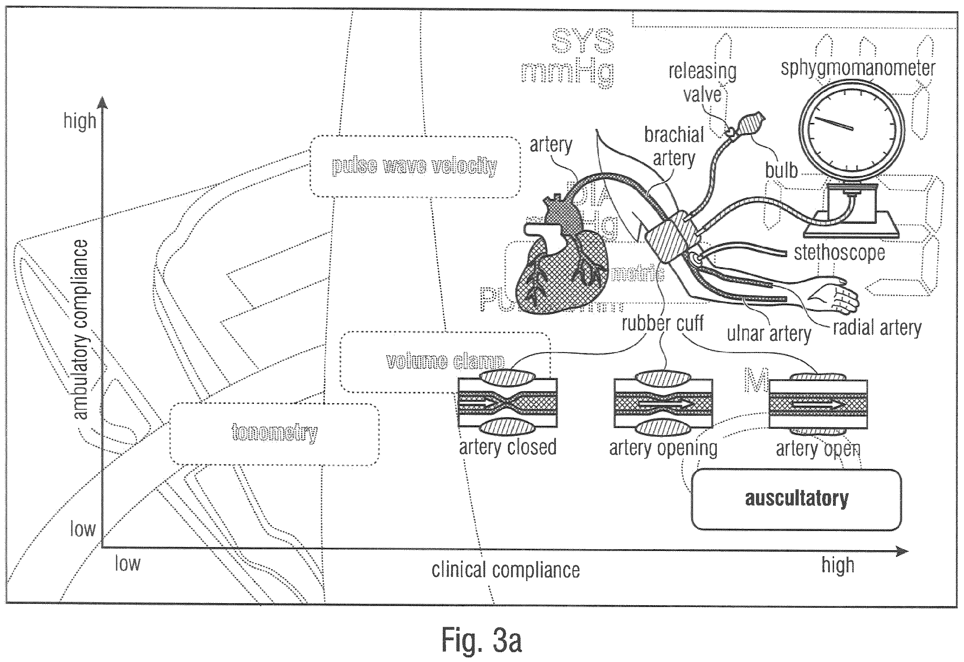

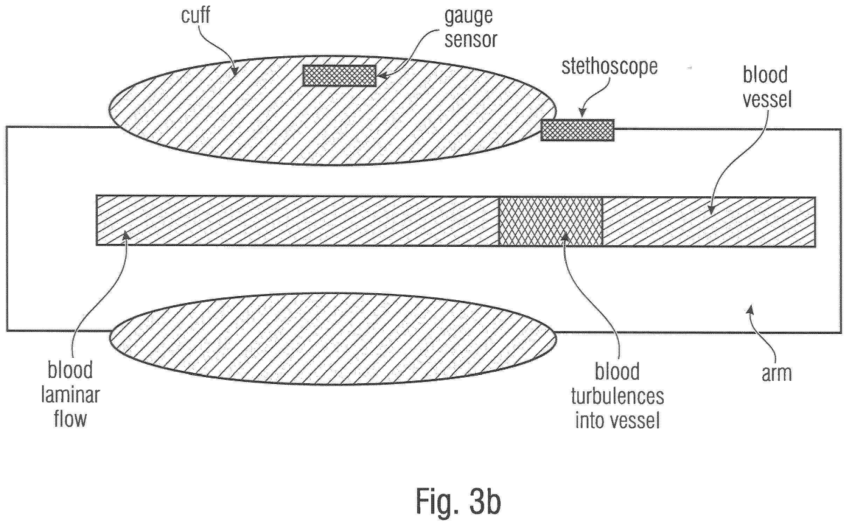

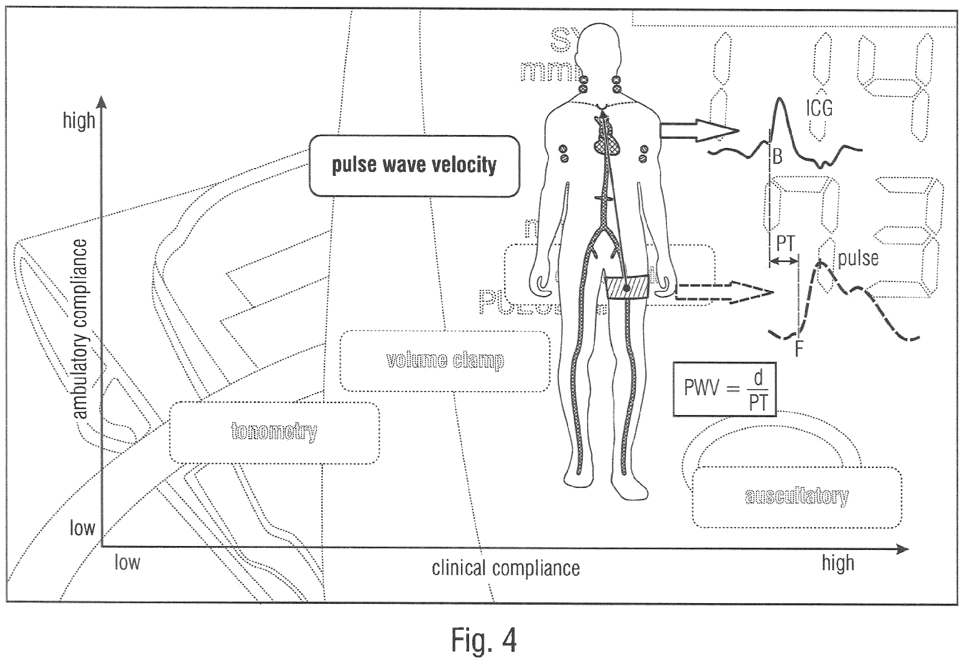

All known non-invasive blood pressure monitoring methods have pros and cons specially related to accuracy, occlusiveness (e.g., occlusive, semi-occlusive or none-occlusive nature of the method), portability/ambulatory and form factor, see Fig. 1. Today, there exist Sphygmomanometer based on an oscillometric method, see Figs. 1, 2a and 2b, or an auscultatory method, see Figs. 1, 3a and 3b. Both methods are accurate and occlusive type. Due to the methods being of the occlusive type, they are not compatible with continuous blood pressure monitoring. After a couple of hours, the accuracy decreases and the discomfort of the patient increases. Oscillometric methods are also not suitable for over-night monitoring, since the periodic inflation of the cuff induces frequent awakenings from sleep. Pulse wave velocity is non-occlusive, but sensitive to arm movement and needs calibration on wrist, see Figs. 1 and 4. For the Pulse Wave Velocity (PWV) method exist different implementations combining optical, ultrasonic or radar with an electrocardiogram. A tonometric method is semi-occlusive mainly for mean artery pressure monitoring and known to be less accurate but suitable for some use cases. No method was acceptable from accuracy point of view and also able to cover different use cases like continuous monitoring during sleep, during office activities or during outdoor activities like cycling. Blood pressure (BP) values can rapidly change in response to physical activity, emotions and stress. Hence, it is crucial to alert the user to obtain timely interventions.

The document US2014012147A1 relates to methods and apparatuses for continuous non-invasive blood pressure (CNIBP) measurement. The methods and apparatuses monitor features of pulse waves in a subject and initiate recalibration when the monitored features differ from corresponding reference features in a stored template.

Therefore, it is desired to get a concept which improves a blood pressure monitoring accuracy, improves an ambulatory compliance and/or enables a long-term monitoring.

### Summary

It has been realized that one problem encountered when trying to improve blood pressure monitoring devices stems from the fact that occlusive methods are accurate but not suitable for continuous long-term blood pressure monitoring. This difficulty is overcome by determining reference data based on first measurement data obtained in an occlusive or semi-occlusive manner at an initializing of a blood pressure monitoring interval, e.g., to correct second measurement data obtained in a non-occlusive manner during the blood pressure monitoring interval. The inventors found, that it is advantageous to combine an occlusive or semi-occlusive measurement and a non-occlusive measurement in one blood pressure monitoring device and to mainly use the non-occlusive measurement of blood volume changes or pulse wave changes to obtain current measurement data. This is based on the idea that a high accuracy can be achieved for a determination of blood pressure values during the blood pressure monitoring interval, if this determination is based on the second measurement data obtained by non-occlusively measuring blood volume changes and if this determination is based on reference data determined based on first measurement data obtained by occlusively or semi-occlusively measuring blood pressure values. To achieve a high accuracy, it is not necessary to continuously measure blood pressure values in an occlusive or semi-occlusive manner. Instead it is sufficient to obtain the first measurement data by occlusively or semi-occlusively, e.g. oscillometrically or tonometrically, measuring blood pressure values once at the initialization of the blood pressure monitoring interval to determine the reference data and then use this reference data during the blood pressure monitoring interval. Due to the measuring of the blood volume changes being non-occlusive, it is possible to obtain the second measurement data continuously during the blood pressure monitoring interval. Thus, a highly accurate blood pressure monitoring device can be implemented for long-term monitoring.

Accordingly, the invention is best summarized by the appended claims.

An embodiment is related to a computer program having a program code for performing, when running on a computer, a herein described method.

### Brief Description of the Drawings

The drawings are not necessarily to scale; emphasis instead generally being placed upon illustrating the principles of the present application. In the following description, various embodiments of the present application are described with reference to the following drawings, in which:
- Fig. 1: shows known blood pressure monitoring methods;
- Fig. 2a: shows diagrams illustrating an oscillometric method;
- Fig. 2b: shows schematically an application of an oscillometric method;
- Fig. 3a: shows an auscultatory method in detail;
- Fig. 3b: shows schematically an application of an auscultatory method;
- Fig. 4: shows a pulse wave measurement;
- Fig. 5: shows schematically a blood pressure monitoring device, according to an embodiment;
- Fig. 6: shows schematically details of a controller of a blood pressure monitoring device, according to an embodiment;
- Fig. 7: shows schematically a blood pressure monitoring device with an inflatable cuff, according to an embodiment;
- Fig. 8: shows diagrams illustrating the principle of an oscillometric method;
- Fig. 9: shows schematically a blood pressure monitoring device with two inflatable cuffs, according to an embodiment;
- Fig. 10: shows schematically an adaptive blood pressure monitoring device, according to an embodiment;
- Fig. 11: shows schematically a blood pressure monitoring device with five sensors, according to an embodiment;
- Fig. 12: shows a block diagram of a method for blood pressure monitoring, according to an embodiment;
- Fig. 13: shows a predetermined position of a blood pressure monitoring device during calibration, according to an embodiment; and
- Fig. 14: shows a diagram illustrating advantages of a blood pressure monitoring device, according to an embodiment, with respect to known implementations.

### Detailed Description of the Embodiments

Equal or equivalent elements or elements with equal or equivalent functionality are denoted in the following description by equal or equivalent reference numerals even if occurring in different figures.

In the following description, a plurality of details is set forth to provide a more throughout explanation of embodiments of the present invention. However, it will be apparent to those skilled in the art that embodiments of the present invention may be practiced without these specific details. In other instances, well-known structures and devices are shown in block diagram form rather than in detail in order to avoid obscuring embodiments of the present invention. In addition, features of the different embodiments described herein after may be combined with each other, unless specifically noted otherwise.

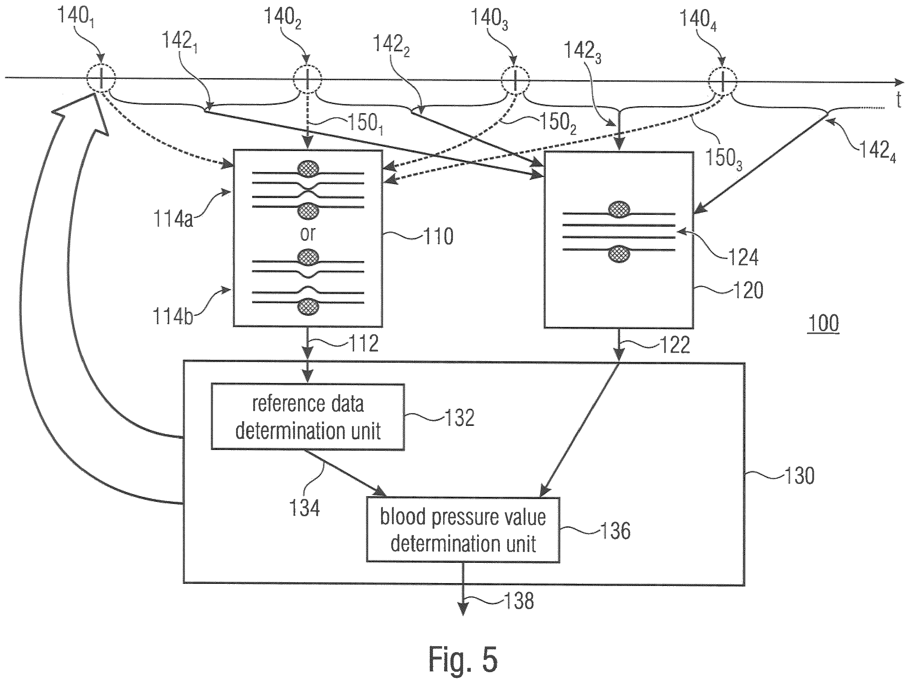

Fig. 5 shows a blood pressure monitoring device 100, comprising a first sensor 110, a second sensor 120 and a controller 130. This monitoring device is not in accordance with the claimed invention, but merely described since it is useful to understand the invention.

The first sensor 110 is configured to obtain first measurement data 112 by occlusively 114a or semi-occlusively 114b measuring blood pressure values, e.g., at an extremity of a person and/or mammal, and the second sensor 120 is configured to obtain second measurement data 122 by non-occlusively measuring 124 blood volume changes, e.g., pulse wave changes based on blood volume changes, e.g., in a body of the person and/or mammal.

The first sensor 110 might be a gauge pressure sensor. Thus, a high SNR (signal to noise ratio) can be achieved and the blood pressure monitoring device 100 might achieve a performance of an auscultatory method.

The first sensor 110 might be configured to obtain the first measurement data 112 by semi-occlusively 114b, tonometrically measuring the blood pressure values, i.e. by semi-occlusively 114b measuring, in a tonometric manner. The first sensor 110, for example, is configured to obtain the first measurement data 112 by semi-occlusively 114b and tonometrically measuring the blood pressure values. The first sensor 110 might be integrated in an inflatable cuff, so that the first sensor 110 can be pressed against tissue of a person or mammal, during the measurement, without causing occlusion.

Alternatively, or additionally, the first sensor 110 might be configured to obtain the first measurement data 112 by occlusively 114a, oscillometrically measuring the blood pressure values, i.e. by occlusively 114a measuring, in an oscillometric manner. The first sensor 110, for example, is configured to obtain the first measurement data 112 by occlusively 114a and oscillometrically measuring the blood pressure values.

The second sensor 120 might be a photoplethysmographic sensor. According to an embodiment, the second sensor 120 is configured to measure 124 the blood volume changes using light (opto), radar or ultrasound. Optionally, the second sensor 120 is configured to measure 124 the blood volume changes, additionally or alternatively, by measuring an electrocardiogram.

In case of the second sensor 120 being a photoplethysmographic sensor, the controller 130 might also be configured to determine heart rate estimates. When a conventional dual-wavelength photoplethysmographic sensor is used as second sensor 120, oxygen saturation might be extracted as additional monitoring parameter.

According to an embodiment, the second sensor represents a sensor unit comprising two or more sensors. In case the sensor unit, e.g., comprises a photoplethysmographic sensor and a sensor configured to measure an electrocardiogram, the controller might be configured to obtain a pulse wave velocity as the second measurement data.

The controller 130, i.e. a processing means, is configured to initialize 140, see 140₁ to 140₄, and conduct a blood pressure monitoring interval 142, see 142, to 142₄. When initializing 140 the blood pressure monitoring interval 142, the controller 130 is configured to achieve the first measurement data 112 from the first sensor 110 and to determine 132 reference data 134 for the second sensor 120 based on the first measurement data 112, e.g., using a reference data determination unit. During the blood pressure monitoring interval 142, the controller 130 is configured to achieve the second measurement data 122 from the second sensor 120. Additionally, the controller 130 is configured to determine 136 blood pressure values 138 during the blood pressure monitoring interval 142 based on the second measurement data 122 and the reference data 134, e.g., using a blood pressure value determination unit.

The controller 130, for example, is configured to initialize 140 the blood pressure monitoring interval 142 by instructing the first sensor 110 to obtain the first measurement data 112 and optionally by instructing the second sensor 120 to start a sequence of pulse wave changes measurements or blood volume changes measurements. According to an embodiment, the controller 130 enables, at the initialization 140 of the blood pressure monitoring interval 142, the first sensor 110 to occulsively 114a or semi-occulsively 114b measure the blood pressure values during an initialization time interval. Optionally, the controller 130 enables the second sensor 120 to start obtaining the second measurement data 122 parallel to the first sensor 110 and to continue obtaining the second measurement data 122 after an end of the initialization time interval. According to an alternative option, the controller 130 enables the second sensor 120 to start obtaining the second measurement data 122 at the end of the initialization time interval.

The blood pressure monitoring interval 142 might be understood as a blood pressure monitoring sequence, e.g., conducted by the second sensor 120 to obtain the second measurement data 122. The controller 130, for example, is configured to conduct the blood pressure monitoring interval 142 by instructing the second sensor 120 to conduct a sequence, i.e. the blood pressure monitoring sequence, of non-occlusive measurements, e.g., of pulse wave changes or of blood volume changes, to obtain the second data 122. The sequence of non-occlusive measurements or a duration of the sequence of non-occlusive measurements might define the blood pressure monitoring interval 142. The second measurement data 122 might represent real time data.

According to an embodiment, the reference data 134 might be used by the controller 130 for verifying the second measurement data 122.

According to an embodiment, the reference data 134 determined 132 by the controller 130 might be understood as calibration data. The second measurement data 122 might be rendered, e.g. corrected, based on the reference data 134. The controller 130 might be configured to use the reference data 134 to calibrate the second sensor 120.

According to an embodiment, the controller 130 might be configured to model an algorithm using the reference data 134 and use the modeled algorithm to render the second measurement data 122 to determine 136 the blood pressure values 138, e.g., to estimate the blood pressure values 138.

According to an embodiment, the controller 130 is configured to update 150, see 150₁ to 150₃, the reference data 134 when initializing 140₂ to 140₄ a new blood pressure monitoring interval 142₂ to 142₄ by achieving new first measurement data 112 from the first sensor 110 and by determining 132 the reference data 134 for the second sensor 120 based on the new first measurement data 112. The controller 130 might instruct/trigger the first sensor 110 to obtain the new first measurement data 112. The controller might be configured to determine 132, for each of consecutive blood pressure monitoring intervals 142, the reference data 134 based on first measurement data 112 obtained when initializing the respective blood pressure monitoring interval 142. The reference data 134 obtained for the individual blood pressure monitoring intervals 142 might differ among each other. However, it is possible that reference data 134 of two or more blood pressure monitoring intervals 142 might be equal to each other.

According to an embodiment, the controller 130 is configured to trigger the initialization 140 of the new blood pressure monitoring interval 142 periodically, as shown in Fig. 5. A time duration of a blood pressure monitoring interval 142 might be pre-defined, e.g. 1 hour, 1 day or 1 week, and the controller 130 might be configured to trigger the initialization 140 of the new blood pressure monitoring interval at an end of a current blood pressure monitoring interval, e.g., a second blood pressure monitoring interval 142₂ is initialized 140₂ directly at the end of a first blood pressure monitoring interval 142₁.

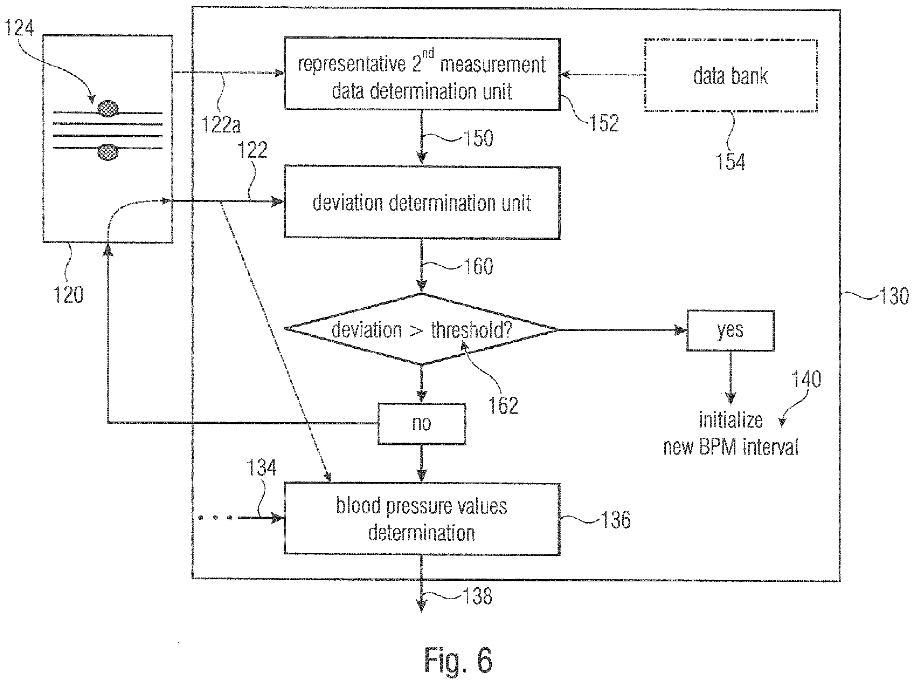

According to an alternative embodiment, shown in Fig. 6, the controller 130 might be configured to obtain representative second measurement data 150, e.g., using a representative second measurement data determination unit 152 and trigger the initialization 140 of the new blood pressure monitoring interval 142 in case of a deviation 160, between current second measurement data 122 obtained from the second sensor 120 during the current blood pressure monitoring interval 142 and the representative second measurement data 150, exceeding a predetermined threshold 162.

The controller 130, e.g., is configured to obtain the representative second measurement data 150 out of a data bank 154.

Alternatively, the controller 130 might be configured to determine, based on second measurement data 122a, e.g. reference second measurement data, obtained from the second sensor 120, the representative second measurement data 150. According to an embodiment, the controller 130 is configured to achieve the second measurement data 122a from the second sensor 120 during the initialization time interval described above. The controller 130, for example, is configured to determine a mean of the second measurement data 122a as the representative second measurement data 150. Optionally, the controller 130 is configured to render the second measurement data 122a using the reference data 134 to obtain the representative second measurement data 150.

The representative second measurement data 150 might be determined/obtained once for a sequence of multiple blood pressure monitoring intervals 142 or for each of consecutive blood pressure monitoring intervals 142.

The controller 130 might be configured to determine the deviation 160, between the current second measurement data 122 obtained from the second sensor 120 during the current blood pressure monitoring interval 142 and the representative second measurement data 150. In case of the deviation 160 not exceeding the predetermined threshold 162, the controller 130 is configured to continue conducting the current blood pressure monitoring interval 142, e.g., by determining 136 the blood pressure values 138 and achieving further second measurement data 122 from the second sensor 120. In case of the deviation 160 exceeding the predetermined threshold 162, the controller 130 is configured to initialize 140 a new blood pressure monitoring interval 142.

The predetermined threshold 162 might define a deviation of +/-5%, +/-10% or +/-15% from the representative second measurement data 150.

In other words, the controller 130 of a blood pressure monitoring device 100, see Fig. 5, might be configured to initialize 140 a new blood pressure monitoring interval 142 in an alert situation, e.g., in case of too much drift of the second measurement data 122. The predetermined threshold 162 might indicate a maximal drift of the second measurement data 122. Thus, the controller is configured to intermittently, e.g., sporadically, trigger the first sensor 110 for achieving the first measurement data, i.e. only in case of initialization of a new blood pressure monitoring interval 142. A duration of a blood pressure monitoring interval 142 is thus given by the time from initializing the blood pressure monitoring interval 142 until initializing a new blood pressure monitoring interval 142 due to a deviation exceeding the predetermined threshold 162. Thus, consecutive blood pressure monitoring intervals 142 might have different durations.

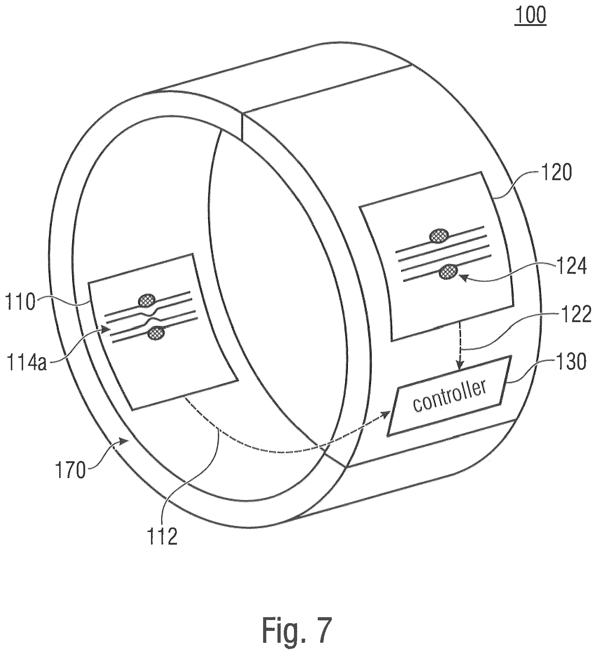

Fig. 7 shows a schematic embodiment of a blood pressure monitoring device 100, which can comprise features and/or functionalities as described with regard to Figs. 5 and/or 6. This monitoring device is not in accordance with the claimed invention, but merely described since it is useful for understanding the invention.

The blood pressure monitoring device 100, shown in Fig. 7, comprises a first inflatable cuff 170 comprising the first sensor 110.

The first sensor 110 is configured to obtain first measurement data 112 by occlusively 114a, oscillometrically measuring the blood pressure values, i.e. by occlusively 114a and oscillometrically measuring the blood pressure values. In other words, the first sensor 110 might be configured to obtain the first measurement data 112 by occlusively 114a measuring, in an oscillometric manner, the blood pressure values.

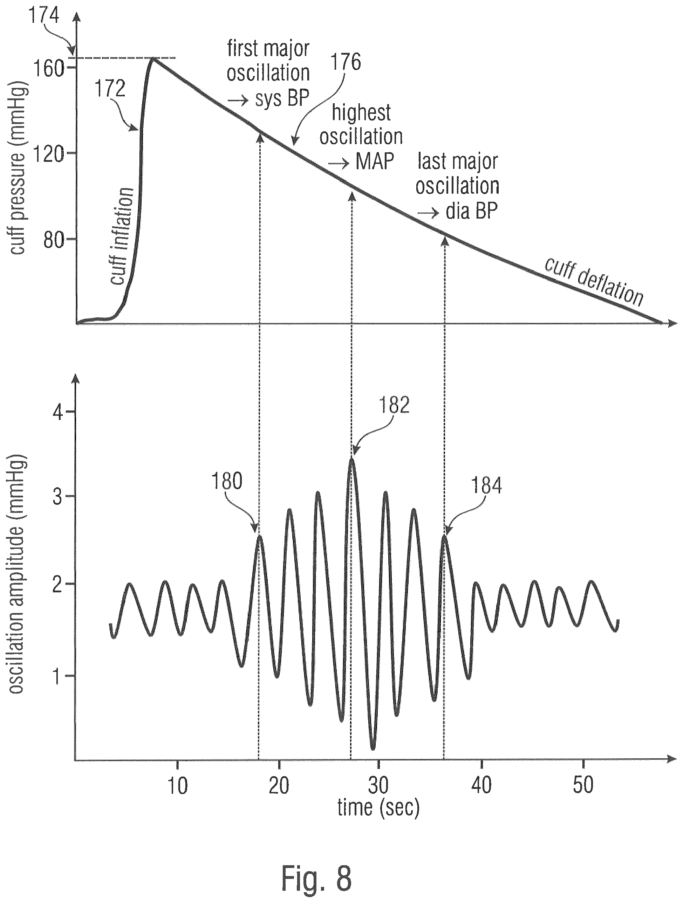

According to an embodiment, the controller 130 is configured to conduct the occlusively 114a, oscillometrically measuring of blood pressure values by inflating 172 the first inflatable cuff 170 up to a predetermined pressure 174, and venting 176 gradually the first inflatable cuff 170 and instructing the first sensor 110 to simultaneously measure oscillations in cuff-pressure, i.e. an oscillation pressure pattern, to obtain the first measurement data 112, see Fig. 7 and Fig. 8. In other words, the first sensor 110₁ is activated by the controller parallel to the venting 176, i.e. the controller 130 is configured to instruct the first sensor 110 to simultaneously to the venting 176 measure oscillations in cuff-pressure.

As shown in Fig. 8 (Source: https://doi.org/10.1371/journal.pone.0216538.g001), the oscillation pressure pattern reflects typical deduction points in blood pressure (BP) measurements. With the first major oscillation 180 marking the systolic (sys) BP, the amplitude further rises until the maximum peak 182 (mean arterial pressure, MAP). The following decrease ends with the last major oscillation amplitude 184 as the diastolic (dia) BP point.

Returning back to Fig. 7, the controller 130 is, for example, configured to achieve the first measurement data 112 from the first sensor 110 and to determine the reference data for the second sensor 120 based on at least two measurement points of the measured oscillations in cuff-pressure, e.g., based on the first major oscillation 180 marking the systolic blood pressure value and based on the last major oscillation amplitude 184 marking the diastolic blood pressure value, as shown in Fig. 8. This enables a calibration of an offset and a sensitivity for systolic and diastolic blood pressures for the second sensor 120.

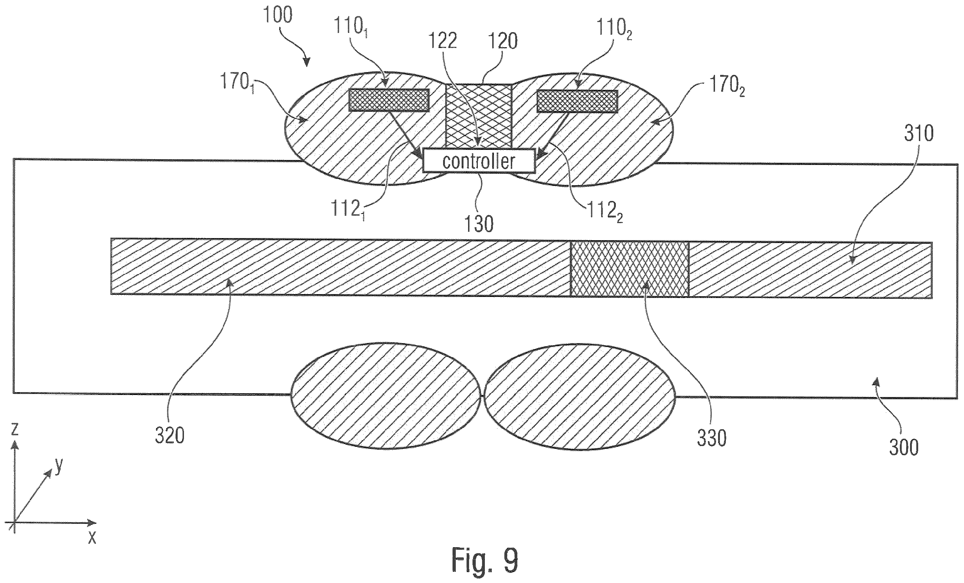

Fig. 9 shows a schematic embodiment of an alternative blood pressure monitoring device 100, which is according to the invention and comprises additionally to the first inflatable cuff 170, comprising the first sensor 110₁ and the second sensor 120, as shown in Fig. 7, a third sensor 110₂ configured to obtain third measurement data 112₂ by occlusively 114a, oscillometrically measuring blood pressure values, and a second inflatable cuff 170₂ comprising the third sensor 110₂. The blood pressure monitoring device 100, shown in Fig. 9, might comprise features and/or functionalities as described with regard to Figs. 5 to 8.

The controller 130 is configured to achieve the first measurement data 112₁ from the first sensor 110₁ and to achieve the third measurement data 112₂ from the third sensor 112₂ and to determine 132 the reference data 134 for the second sensor 120 based on the first measurement data 112₁ and the third measurement data 112₂ when initializing 140 the blood pressure monitoring interval 142. The controller 130 might enable, at the initialization 140 of the blood pressure monitoring interval 142, the first sensor 110₁ and the third sensor 110₂ to occlusively 114a, oscillometrically measure 114 the blood pressure values during an initialization time interval.

According to an embodiment, the controller 130, shown in Fig. 9, is configured to conduct the occlusively 114a, oscillometrically measuring of blood pressure values by simultaneously inflating 172 the first inflatable cuff 170₁ and the second inflatable cuff 170₂ up to a predetermined pressure 174, and
simultaneously venting 176 gradually the first inflatable cuff 170₁ and the second inflatable cuff 170₂ and instructing the first sensor 110₁ and the third sensor 110₂ to simultaneously, e.g., simultaneously to the venting 176, measure oscillations in cuff-pressure to obtain the first measurement data 112₁ and the third measurement data 112₂, compare Fig. 8. In other words, the first inflatable cuff 170₁ and the second inflatable cuff 170₂ are vented parallel, at the same time, and the first sensor 110₁ and the third sensor 110₂ are activated by the controller parallel to the venting 176.

According to an embodiment, the controller 130, shown in Fig. 9, is configured to achieve the first measurement data 112₁ from the first sensor 110₁ and to achieve the third measurement data 112₂ from the third sensor 110₂ and to determine 132 the reference data 134 for the second sensor 120 based on at least two measurement points of the measured oscillations in cuff-pressure of the first measurement data 112₁ and based on at least two measurement points of the measured oscillations in cuff-pressure of the third measurement data 112₂ when initializing the blood pressure monitoring interval. For example, the first major oscillation 180 marking the systolic blood pressure value and the last major oscillation amplitude 184 marking the diastolic blood pressure value, are considered as two measurement points, compare Fig. 8. This enables a calibration of an offset and a sensitivity for systolic and diastolic blood pressures for the second sensor 120.

As shown in Fig. 9, the second sensor 120 is positioned laterally between the first inflatable cuff 170₁ and the second inflatable cuff 170₂. This means that the first inflatable cuff 170₁, the second sensor 120 and the second inflatable cuff 170₂ are arranged in this order next to each other in the same plane, e.g., an xy-plane. The first inflatable cuff 170₁, for example, is positioned on one side, e.g., one the right side, of the second sensor 120 and the second inflatable cuff 170₂ is positioned on an opposite side, e.g., on the left side, of the second sensor 120.

According to an embodiment, the first sensor 110₁ and/or the third sensor 110₂ are/is a gauge pressure sensor.

The two cuffs, i.e. the first inflatable cuff 170₁ and the second inflatable cuff 170₂, three sensors, i.e. the first sensor 110₁, the second sensor 120 and the third sensor 110₂, solution will differentiate between muscles/ligament movement and blood pressure waves and improve accuracy of BPM, e.g., by suppressing pressure spikes due to muscle movements.

The blood pressure monitoring device 100 composes of a main sensor, i.e. the second sensor 120 (photoplethysmographic sensor, radar, ultrasounds or ECG), able to measure blood volume changes, surrounded by a couple of inflatable cuffs 170₁ and 170₂, able to measure pressure changes. The former might enable continuous non-obstructive monitoring, while the latter might be responsible for obtaining reference calibration data, i.e. the reference data 134.

Specifically, the pair of cuffs 170₁ and 170₂ replicates an occlusive 114a measuring, in an oscillometric manner, for accurate BP estimate. The whole ABPM (adaptive blood pressure monitoring) system, i.e. the blood pressure monitoring device 100, uses the data measured from the oscillometric method (ground truth) to tune parameters of an algorithm responsible for returning a continuous beat-to-beat BP estimate from the second measurement data 122 obtained by the non-obstructive method, i.e. by the central sensor, the second sensor 120. Doing so, the accuracy of the oscillometric method is mapped into the continuous measurement for reliable and real-time information about patient conditions.

In Fig. 9, the blood pressure monitoring device 100, for example, is illustrated at an arm 300 with a blood vessel 310. Inside the blood vessel 310, for example, exists laminar flow 320 of blood and blood turbulences 330. It is to be noted that alternatively the blood pressure monitoring device 100 can be positioned at a different position of a person or mammal, e.g., at a different extremity of a person or mammal.

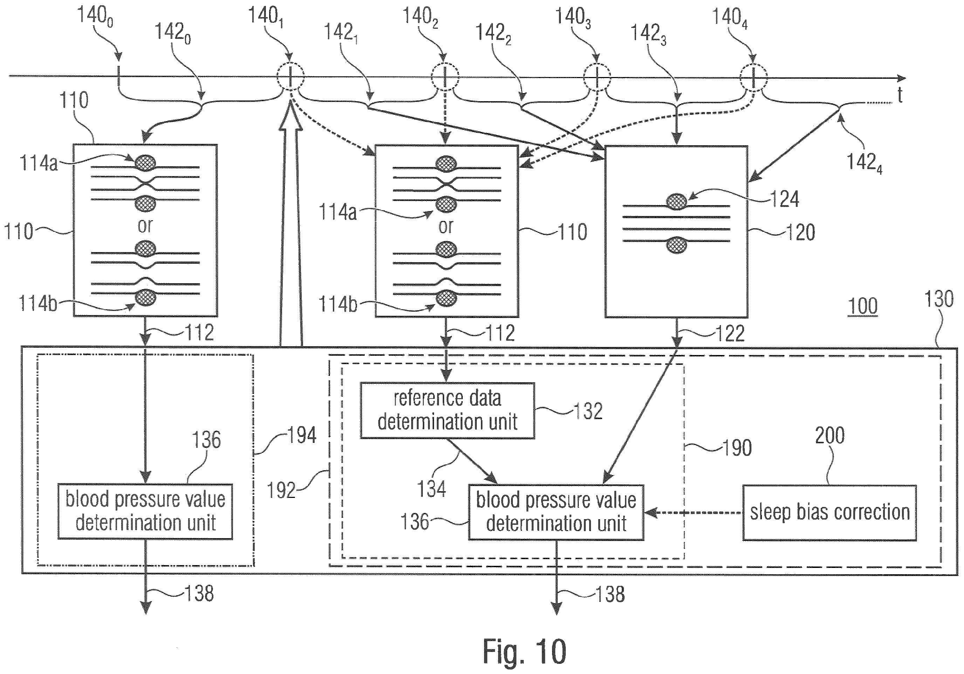

According to an embodiment, shown in Fig. 10, the blood pressure monitoring device 100 might be configured to work in two or more modes. This implementation might be used by any aforementioned blood pressure monitoring device 100.

Additionally, to a normal mode 190 described above, the blood pressure monitoring device 100 is optionally configured to work in a sleep mode 192. The controller 130 is configured to determine 136, in the sleep mode 192, the blood pressure values 138 during the blood pressure monitoring interval 142 based on the second measurement data 120 and the reference data 134 using a processing means 200 to correct a sleep bias. The processing means 200, e.g., is at least partially implemented in software, e.g. representing an algorithm. The processing means 200 might be configured to increase blood pressure values, e.g., to compensate for a subsidence of blood pressure values due to a sleeping position of a person or mammal using the blood pressure monitoring device 100. Thus, a self-calibration PWV BP for sleeping mode is provided.

During sleeping position, the blood pressure estimates, i.e. the blood pressure values 138, do not provide typical results. The body is elevated and the resulting estimates are lowered. Nevertheless, this estimated bias can be modelled and, thus, corrected via the estimate algorithms, i.e. the processing means 200.

Additionally to the normal mode 190 and optionally additionally to the sleep mode 192, the blood pressure monitoring device 100 is optionally configured to work in a MAP (mean arterial pressure) mode 194, wherein the controller 130 is configured achieve, in the MAP mode 194, the first measurement data 112 from the first sensor 110 during the blood pressure monitoring interval 142₀ and determine 136 blood pressure values 138 during the blood pressure monitoring interval 142₀ based on the first measurement data 112. Thus, in the MAP mode 194, the first sensor 110 is activated during the complete blood pressure monitoring interval 142₀ and not only at the initialization time interval, as for the normal mode 190 and the sleep mode 192.

In the MAP mode 194, the second sensor 120 might be deactivated during the blood pressure monitoring interval 142₀ or might be additionally activated parallel to the first sensor 110 to improve the accuracy of the determined blood pressure values 138. In case of an activated second sensor 120, the controller 130 might be configured to determine 136 the blood pressure values 138 during the blood pressure monitoring interval 142₀ based on the first measurement data 112 and the second measurement data 122.

According to an embodiment, the controller 130 is configured to determine 136, in the MAP mode 194, systolic and diastolic blood pressure values based on the first measurement data 112. The systolic and diastolic blood pressure values can be extracted by algorithm processing steps.

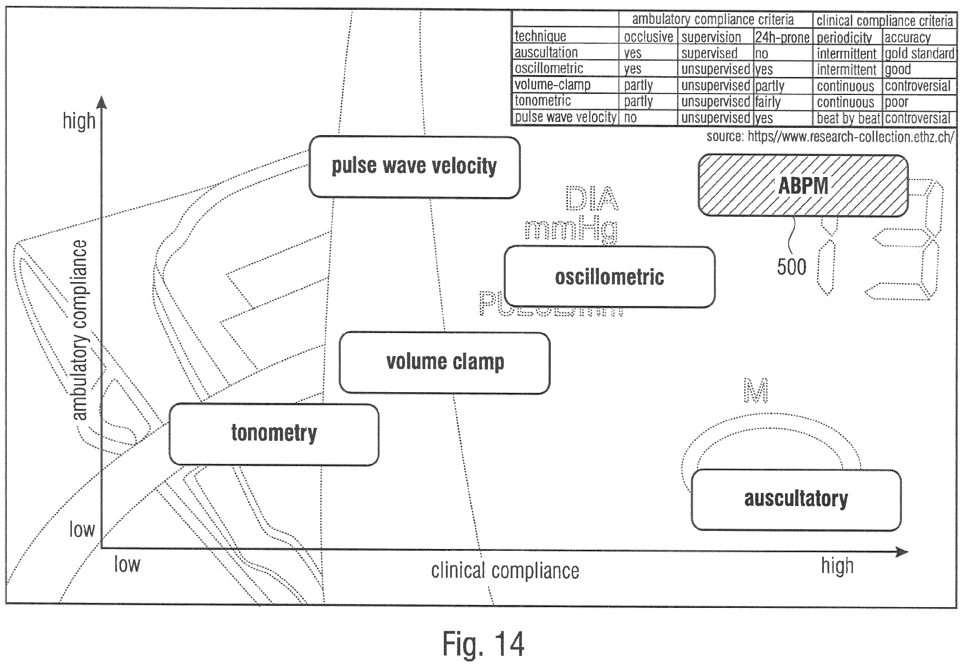

The blood pressure monitoring device 100, shown in Fig. 10, enables an adaptive and non-invasive continuous blood pressure monitoring, e.g., depending on a situation in which the blood pressure monitoring device 100 is used, the best mode, i.e. the normal mode 190, the sleep mode 192 or the MAP mode 194, for the respective situation can be selected and executed by the blood pressure monitoring device 100. As shown in Fig. 14, such an adaptive and non-invasive continuous blood pressure monitoring 500 is advantageous compared to known blood pressure monitoring methods due to a high clinical compliance and a high ambulatory compliance.

The system can be set in sleeping BP monitor, i.e. in sleep mode 192, when only non-occlusive BPM (blood pressure monitoring) is acceptable. For outdoor activities like hiking or cycling the ABPM can be set as non-occlusive mode and monitor MAP by using oscillometric method, i.e. for outdoor activities it is possible to choose between the normal mode 190 and the MAP mode 194.

Although Fig. 10 shows an own first sensor 110 for the MAP mode, this is only for illustration purposes to prevent an obscuring illustration of the principle and it is clear that the measurement is performed by the same first sensor 110 illustrated for the normal mode 190 and the sleep mode 192.

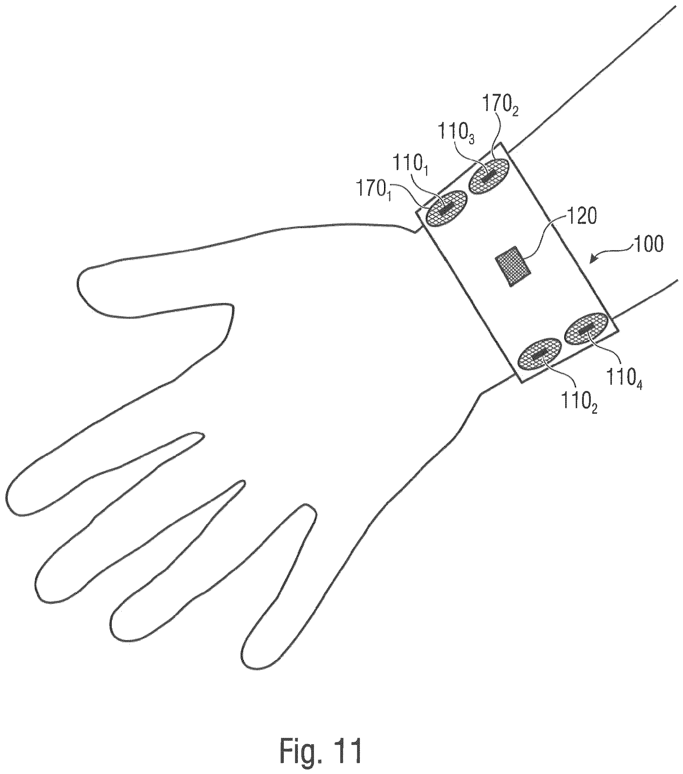

Fig. 11, shows an embodiment of the blood pressure monitoring device 100 with the first inflatable cuff 170₁ and the second inflatable cuff 170₂ differing from the blood pressure monitoring device 100, shown in Fig. 9, by comprising four sensors 110₁ to 110₄ configured to obtain measurement data by occlusively 114a, oscillometrically measuring blood pressure values, based on which measurement data the controller 130 (not shown in Fig. 11) is configured to determine 132 the reference data 134. Each of the two inflatable cuffs 170 comprises two of the sensors 110, e.g., the first inflatable cuff 170₁ comprises the sensors 110₁ and 110₂ and the second inflatable cuff 170₂ comprises the sensors 110₃ and 110₄. The second sensor 120 is positioned laterally between the first inflatable cuff 170₁ and the second inflatable cuff 170₂, i.e. surrounded by the sensors 110.

The blood pressure monitoring device 100 presented herein might be for a non-invasive blood pressure monitoring at an extremity of a person or mammal. This might be realized by a wearable, e.g., a wristband or a watch, comprising the blood pressure monitoring device 100. This is due to the small form factor and a low power consumption, whereby the blood pressure monitoring device 100 is suitable for smartwatch integration.

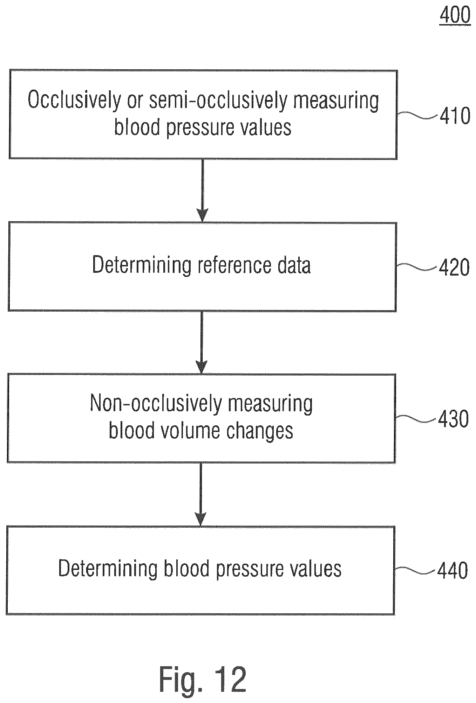

Fig. 12 shows a method 400 for blood pressure monitoring, comprising occlusively or semi-occlusively measuring 410 blood pressure values using a first sensor to obtain first measurement data and non-occlusively measuring 430 blood volume changes using a second sensor to obtain second measurement data. The method 400 comprises initializing and conducting a blood pressure monitoring interval by achieving the first measurement data from the first sensor and determining 420 reference data for the second sensor based on the first measurement data when initializing the blood pressure monitoring interval, achieving the second measurement data from the second sensor during the blood pressure monitoring interval, and determining 440 blood pressure values during the blood pressure monitoring interval based on the second measurement data and the reference data.

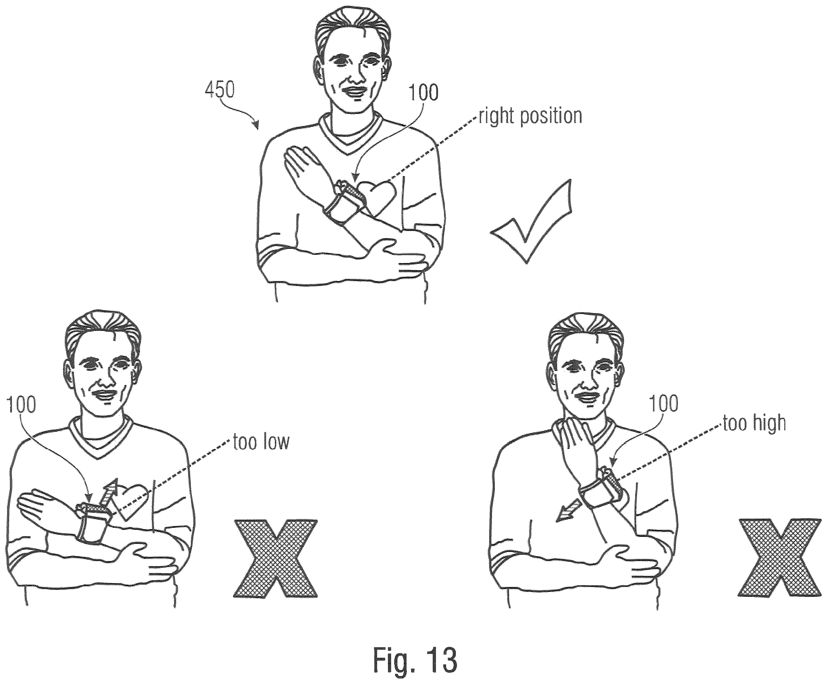

Optionally, the method 400 comprises using a herein described blood pressure monitoring device 100 comprising the first sensor 110 and the second sensor 120, wherein the method 400 comprises positioning the blood pressure monitoring device 100 in a predetermined position 450 at an initializing of the blood pressure monitoring interval, e.g., as shown in Fig. 13. The method 400 might provide instructions via the blood pressure monitoring device 100 to the patient to position the blood pressure monitoring device 100 into the predetermined position 450.

During calibration, i.e. during the initialization time interval, the blood pressure monitoring device 100 is to be placed at the same level as the heart of the patient, i.e. a person or mammal, since blood pressure highly depends on body positions (see Figure 13 for test position). The blood pressure monitoring device 100 and/or the method 400 might trigger periodic reminders when calibration is required, in order to keep the calibration parameters, i.e. the reference data 134 updated while compensating for aging (e.g. optical power reduction) or physiological effects (e.g., changes in BMI).

The calibration might be required at the startup of the blood pressure monitoring device 100 and/or the method 400 as well as every time the system triggers an alarm, see Fig. 6. This will allow recalibration points over time, maintaining the original accuracy.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The inventive algorithm to determine 136 the blood pressure values 138 and/or to perform the sleep bias correction 200 can be stored on a digital storage medium or can be transmitted on a transmission medium such as a wireless transmission medium or a wired transmission medium such as the Internet.

Depending on certain implementation requirements, embodiments of the invention can be implemented in hardware or in software. The implementation can be performed using a digital storage medium, for example a floppy disk, a DVD, a CD, a ROM, a PROM, an EPROM, an EEPROM or a FLASH memory, having electronically readable control signals stored thereon, which cooperate (or are capable of cooperating) with a programmable computer system such that the respective method is performed.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

## Claims

1. Blood pressure monitoring device (100), comprising:
a first sensor (110; 110₁) configured to obtain first measurement data (112; 112₁) by occlusively (114a), oscillometrically measuring blood pressure values,
a second sensor (120) configured to obtain second measurement data (122) by non-occlusively measuring (124) blood volume changes,
a third sensor (110₂) configured to obtain third measurement data (112₂) by occlusively (114a), oscillometrically measuring (114) blood pressure values,
a controller (130) configured to initialize (140) and conduct a blood pressure monitoring interval (142), wherein the controller (130) is configured
- to achieve the first measurement data (112; 112₁) from the first sensor (110; 110₁) and to achieve the third measurement data (112₂) from the third sensor (110₂) and to determine (132) reference data (134) for the second sensor (120) based on the first measurement data (112; 112₁) and the third measurement data (112₂) when initializing (140) the blood pressure monitoring interval (142),
- to achieve the second measurement data (122) from the second sensor (120) during the blood pressure monitoring interval (142), and
- to determine (136) blood pressure values (138) during the blood pressure monitoring interval (142) based on the second measurement data (122) and the reference data (134),
a first inflatable cuff (170; 170₁) comprising the first sensor (110; 110₁), and
a second inflatable cuff (170₂) comprising the third sensor (110₂),
wherein the second sensor (120) is positioned laterally between the first inflatable cuff (170; 170₁) and the second inflatable cuff (170₂).

2. Blood pressure monitoring device (100) according to claim 1, wherein the controller (130) is configured to update the reference data (134) when initializing (140) a new blood pressure monitoring interval (142) by achieving new first measurement data (112; 112₁) from the first sensor (110; 110₁) and by determining the reference data (134) for the second sensor (120) based on the new first measurement data (112; 112₁).

3. Blood pressure monitoring device (100) according to claim 2, wherein the controller (130) is configured to trigger the initialization (140) of the new blood pressure monitoring interval (142) periodically.

4. Blood pressure monitoring device (100) according to claim 2, wherein the controller (130) is configured to obtain representative second measurement data (150), and
initialize (140) the new blood pressure monitoring interval (142) in case of a deviation (160), between current second measurement data (122) obtained from the second sensor (120) during the blood pressure monitoring interval (142) and the representative second measurement data (150), exceeding a predetermined threshold (162).

5. Blood pressure monitoring device (100) according to one of claims 1 to 4, wherein the controller (130) is configured to conduct the occlusively (114a), oscillometrically measuring (114) of blood pressure values by
Inflating (172) the first inflatable cuff (170; 170₁) up to a predetermined pressure (174), and
venting (176) gradually the first inflatable cuff (170; 170₁) and instructing the first sensor (110; 110₁) to simultaneously measure oscillations in cuff-pressure to obtain the first measurement data (112; 112₁).

6. Blood pressure monitoring device (100) according to claim 5, wherein the controller (130) is configured to achieve the first measurement data (112; 112₁) from the first sensor (110; 110₁) and to determine (132) the reference data (134) for the second sensor (120) based on at least two measurement points (180, 184) of the measured oscillations in cuff-pressure.

7. Blood pressure monitoring device (100) according to one of claims 1 to 6, wherein the controller (130) is configured to conduct the occlusively (114a), oscillometrically measuring (114) of blood pressure values by
simultaneously inflating (172) the first inflatable cuff (170; 170₁) and the second inflatable cuff (170₂) up to a predetermined pressure (174), and
simultaneously venting (176) gradually the first inflatable cuff (170; 170₁) and the second inflatable cuff (170₂) and instructing the first sensor (110; 110₁) and the third sensor (110₂) to simultaneously measure oscillations in cuff-pressure to obtain the first measurement data (112; 112₁) and the third measurement data (112₂).

8. Blood pressure monitoring device (100) according to claim 7, wherein the controller (130) is configured to achieve the first measurement data (112; 112₁) from the first sensor (110; 110₁) and to achieve the third measurement data (112₂) from the third sensor (110₂) and to determine (132) the reference data (134) for the second sensor (120) based on at least two measurement points (180, 184) of the measured oscillations in cuff-pressure of the first measurement data (112; 112₁) and based on at least two measurement points (180, 184) of the measured oscillations in cuff-pressure of the third measurement data (112₂) when initializing (140) the blood pressure monitoring interval (142).

9. Blood pressure monitoring device (100) according to one of claims 1 to 8, wherein the first sensor (110; 110₁) and/or the third sensor (110₂) are/is a gauge pressure sensor.

10. Blood pressure monitoring device (100) according to one of claims 1 to 9,
wherein the second sensor (120) is a photoplethysmographic sensor and/or
wherein the second sensor (120) is configured to measure the blood volume changes using radar or ultrasound and/or
wherein the second sensor (120) is configured to measure the blood volume changes by measuring an electrocardiogram.

11. Blood pressure monitoring device (100) according to one of claims 1 to 10, configured to work in a sleep mode (192), wherein the controller (130) is configured to determine, in the sleep mode (192), the blood pressure values during the blood pressure monitoring interval (142) based on the second measurement data (122) and the reference data (134) using a processing means (200) to correct a sleep bias.

12. Blood pressure monitoring device (100) according to one of claims 1 to 11, configured to work in a MAP mode (194), wherein the controller (130) is configured achieve, in the MAP mode (194), the first measurement data (112; 112₁) from the first sensor (110; 110₁) during the blood pressure monitoring interval (142) and to determine (136) blood pressure values (138) during the blood pressure monitoring interval (142) based on the first measurement data (112; 112₁).

13. Blood pressure monitoring device (100) according to claim 12, wherein the controller (130) is configured to determine, in the MAP mode (194), systolic and diastolic blood pressure values based on the first measurement data (112; 112₁).

14. Blood Pressure Monitoring device (100) according to one of claims 1 to 13, for a non-invasive blood pressure monitoring at an extremity of a person.

15. Method (400) for blood pressure monitoring, comprising:
occlusively (114a), oscillometrically measuring (410) blood pressure values using a first sensor (110; 110₁) to obtain first measurement data (112; 112₁),
non-occlusively measuring (430; 124) blood volume changes using a second sensor (120) to obtain second measurement data (122),
occlusively (114a), oscillometrically measuring (114) blood pressure values using a third sensor (110₂) to obtain third measurement data (112₂)
initializing (140) and conducting a blood pressure monitoring interval (142) by
- achieving the first measurement data (112; 112₁) from the first sensor (110; 110₁) and achieving the third measurement data (112₂) from the third sensor (110₂) and determining (420; 132) reference data (134) for the second sensor (120) based on the first measurement data (112; 112₁) and the third measurement data (112₂) when initializing (140) the blood pressure monitoring interval (142),
- achieving the second measurement data (122) from the second sensor (120) during the blood pressure monitoring interval (142), and
- determining (440; 136) blood pressure values during the blood pressure monitoring interval (142) based on the second measurement data (122) and the reference data (134)
wherein the first sensor (110; 110₁) is comprised by a first inflatable cuff (170; 170₁),
wherein the third sensor (110₂) is comprised by a second inflatable cuff (170₂), and
wherein the second sensor (120) is positioned laterally between the first inflatable cuff (170; 170₁) and the second inflatable cuff (170₂).

16. Method (400) of claim 15, using a blood pressure monitoring device (100) comprising the first sensor (110; 110₁) and the second sensor (120), wherein the method (400) comprises positioning the blood pressure monitoring device (100) in a predetermined position (450) at an initializing (140) of the blood pressure monitoring interval (142).

17. A computer program for determining the blood pressure values in the method of one of claims 15 and 16.

## Patentansprüche

1. Blutdrucküberwachungsvorrichtung (100), die folgende Merkmale aufweist:
einen ersten Sensor (110; 110₁), der dazu konfiguriert ist, durch okklusives (114a), oszillometrisches Messen von Blutdruckwerten erste Messdaten (112; 112₁) zu erhalten,
einen zweiten Sensor (120), der dazu konfiguriert ist, durch nicht-okklusives Messen (124) von Blutvolumenänderungen zweite Messdaten (122) zu erhalten,
einen dritten Sensor (110₂), der dazu konfiguriert ist, durch okklusives (114a), oszillometrisches Messen (114) von Blutdruckwerten dritte Messdaten (112₂) zu erhalten,
eine Steuerung (130), die dazu konfiguriert ist, ein Blutdrucküberwachungsintervall (142) zu initialisieren (140) und durchzuführen, wobei die Steuerung (130) konfiguriert ist zum:
Erhalten der ersten Messdaten (112; 112₁) von dem ersten Sensor (110; 110₁) und Erhalten der dritten Messdaten (112₂) von dem dritten Sensor (110₂) und Bestimmen (132) von Referenzdaten (134) für den zweiten Sensor (120) basierend auf den ersten Messdaten (112; 112₁) und den dritten Messdaten (112₂), wenn das Blutdrucküberwachungsintervall (142) initialisiert wird (140),
Erhalten der zweiten Messdaten (122) von dem zweiten Sensor (120) während des Blutdrucküberwachungsintervalls (142) und
Bestimmen (136) von Blutdruckwerten (138) während des Blutdrucküberwachungsintervalls (142) basierend auf den zweiten Messdaten (122) und den Referenzdaten (134),
eine erste aufblasbare Manschette (170; 170₁), die den ersten Sensor (110; 110₁) aufweist und
eine zweite aufblasbare Manschette (170₂), die den dritten Sensor (110₂) aufweist,
wobei der zweite Sensor (120) seitlich zwischen der ersten aufblasbaren Manschette (170; 170₁) und der zweiten aufblasbaren Manschette (170₂) positioniert ist.

2. Blutdrucküberwachungsvorrichtung (100) gemäß Anspruch 1, bei der die Steuerung (130) dazu konfiguriert ist, die Referenzdaten (134) zu aktualisieren, wenn ein neues Blutdrucküberwachungsintervall (142) initialisiert wird (140) durch Erhalten neuer erster Messdaten (112; 112₁) von dem ersten Sensor (110; 110₁) und durch Bestimmen der Referenzdaten (134) für den zweiten Sensor (120) basierend auf den neuen ersten Messdaten (112; 112₁).

3. Blutdrucküberwachungsvorrichtung (100) gemäß Anspruch 2, bei der die Steuerung (130) dazu konfiguriert ist, die Initialisierung (140) des neuen Blutdrucküberwachungsintervalls (142) regelmäßig auszulösen.

4. Blutdrucküberwachungsvorrichtung (100) gemäß Anspruch 2, bei der die Steuerung (130) dazu konfiguriert ist, repräsentative zweite Messdaten (150) zu erhalten und
das neue Blutdrucküberwachungsintervall (142) zu initialisieren (140) im Fall einer Abweichung (160) zwischen aktuellen zweiten Messdaten (122), die von dem zweiten Sensor (120) während des Blutdrucküberwachungsintervalls (142) erhalten werden, und den repräsentativen zweiten Messdaten (150), die einen vorbestimmten Schwellenwert (162) überschreiten.

5. Blutdrucküberwachungsvorrichtung (100) gemäß einem der Ansprüche 1 bis 4, bei der die Steuerung (130) dazu konfiguriert ist, das okklusive (114a), oszillometrische Messen (114) von Blutdruckwerten durchzuführen durch
Aufblasen (172) der ersten aufblasbaren Manschette (170; 170₁) bis zu einem vorbestimmten Druck (174) und
allmähliches Entlüften (176) der ersten aufblasbaren Manschette (170; 170₁) und Anweisen des ersten Sensors (110; 110₁), gleichzeitig Oszillationen in dem Manschettendruck zu messen, um die ersten Messdaten (112; 112₁) zu erhalten.

6. Blutdrucküberwachungsvorrichtung (100) gemäß Anspruch 5, bei der die Steuerung (130) dazu konfiguriert ist, die ersten Messdaten (112; 112₁) von dem ersten Sensor (110; 110₁) zu erhalten und die Referenzdaten (134) für den zweiten Sensor (120) basierend auf zumindest zwei Messpunkten (180, 184) der gemessenen Oszillationen in dem Manschettendruck zu bestimmen (132).

7. Blutdrucküberwachungsvorrichtung (100) gemäß einem der Ansprüche 1 bis 6, bei der die Steuerung (130) dazu konfiguriert ist, das okklusive (114a), oszillometrische Messen (114) von Blutdruckwerten durchzuführen durch
gleichzeitiges Aufblasen (172) der ersten aufblasbaren Manschette (170; 170₁) und der zweiten aufblasbaren Manschette (170₂) bis zu einem vorbestimmten Druck (174) und
gleichzeitiges allmähliches Entlüften (176) der ersten aufblasbaren Manschette (170; 170₁) und der zweiten aufblasbaren Manschette (170₂) und Anweisen des ersten Sensors (110; 110₁) und des dritten Sensors (110₂), gleichzeitig Oszillationen in dem Manschettendruck zu messen, um die ersten Messdaten (112; 112₁) und die dritten Messdaten (112₂) zu erhalten.

8. Blutdrucküberwachungsvorrichtung (100) gemäß Anspruch 7, bei der die Steuerung (130) dazu konfiguriert ist, die ersten Messdaten (112; 112₁) von dem ersten Sensor (110; 110₁) zu erhalten und die dritten Messdaten (112₂) von dem dritten Sensor (110₂) zu erhalten und die Referenzdaten (134) für den zweiten Sensor (120) basierend auf zumindest zwei Messpunkten (180, 184) der gemessenen Oszillationen in einem Manschettendruck der ersten Messdaten (112; 112₁) und basierend auf zumindest zwei Messpunkten (180, 184) der gemessenen Oszillationen in dem Manschettendruck der dritten Messdaten (112₂) zu bestimmen, wenn das Blutdrucküberwachungsintervall (142) initialisiert wird (140).

9. Blutdrucküberwachungsvorrichtung (100) gemäß einem der Ansprüche 1 bis 8, bei der der erste Sensor (110; 110₁) und/oder der dritte Sensor (110₂) ein Überdrucksensor ist.

10. Blutdrucküberwachungsvorrichtung (100) gemäß einem der Ansprüche 1 bis 9,
bei der der zweite Sensor (120) ein photoplethysmografischer Sensor ist und/oder
wobei der zweite Sensor (120) dazu konfiguriert ist, die Blutvolumenänderungen unter Verwendung von Radar oder Ultraschall zu messen und/oder
wobei der zweite Sensor (120) dazu konfiguriert ist, die Blutvolumenänderungen durch Messen eines Elektrokardiogramms zu messen.

11. Blutdrucküberwachungsvorrichtung (100) gemäß einem der Ansprüche 1 bis 10, die dazu konfiguriert ist, in einem Schlafmodus (192) zu arbeiten, wobei die Steuerung (130) dazu konfiguriert ist, in dem Schlafmodus (192) die Blutdruckwerte während des Blutdrucküberwachungsintervalls (142) basierend auf den zweiten Messdaten (122) und den Referenzdaten (134) zu bestimmen, unter Verwendung einer Verarbeitungseinrichtung (200), um eine Schlafverzerrung zu korrigieren.

12. Blutdrucküberwachungsvorrichtung (100) gemäß einem der Ansprüche 1 bis 11, die dazu konfiguriert ist, in einem MAD-Modus (194) zu arbeiten, wobei die Steuerung (130) dazu konfiguriert ist, in dem MAD-Modus (194) die ersten Messdaten (112; 112₁) von dem ersten Sensor (110; 110₁) während des Blutdrucküberwachungsintervalls (142) zu erhalten und Blutdruckwerte (138) während des Blutdrucküberwachungsintervalls (142) basierend auf den ersten Messdaten (112; 112₁) zu bestimmen (136).

13. Blutdrucküberwachungsvorrichtung (100) gemäß Anspruch 12, bei der die Steuerung (130) dazu konfiguriert ist, in dem MAD-Modus (194) basierend auf den ersten Messdaten (112; 112₁) systolische und diastolische Blutdruckwerte zu bestimmen.

14. Blutdrucküberwachungsvorrichtung (100) gemäß einem der Ansprüche 1 bis 13 für eine nicht-invasive Blutdrucküberwachung an einer Extremität einer Person.

15. Verfahren (400) zum Blutdrucküberwachen, das folgende Schritte aufweist:
okklusives (114a), oszillometrisches Messen (140) von Blutdruckwerten unter Verwendung eines ersten Sensors (110; 110₁), um erste Messdaten (112; 112₁) zu erhalten,
nicht-okklusives Messen (430; 124) von Blutvolumenänderungen unter Verwendung eines zweiten Sensors (120), um zweite Messdaten (122) zu erhalten,
okklusives (114a), oszillometrisches Messen (114) von den Blutdruckwerten unter Verwendung eines dritten Sensors (110₂), um dritte Messdaten (112₂) zu erhalten,
Initialisieren (140) und Durchführen eines Blutdrucküberwachungsintervalls (142) durch
Erhalten der ersten Messdaten (112; 112₁) von dem ersten Sensor (110; 110₁) und Erhalten der dritten Messdaten (112₂) von dem dritten Sensor (110₂) und Bestimmen (132) von Referenzdaten (134) für den zweiten Sensor (120) basierend auf den ersten Messdaten (112; 112₁) und den dritten Messdaten (112₂), wenn das Blutdrucküberwachungsintervall (142) initialisiert wird (140),
Erhalten der zweiten Messdaten (122) von dem zweiten Sensor (120) während des Blutdrucküberwachungsintervalls (142) und
Bestimmen (136) von Blutdruckwerten (138) während des Blutdrucküberwachungsintervalls (142) basierend auf den zweiten Messdaten (122) und den Referenzdaten (134),
wobei der erste Sensor (110; 110₁) in einer ersten aufblasbaren Manschette (170; 170₁) enthalten ist,
wobei der dritte Sensor (110₂) in einer zweiten aufblasbaren Manschette (170₂) enthalten ist und
wobei der zweite Sensor (120) seitlich zwischen der ersten aufblasbaren Manschette (170; 170₁) und der zweiten aufblasbaren Manschette (170₂) positioniert ist.

16. Verfahren (400) gemäß Anspruch 15, das eine Blutdrucküberwachungsvorrichtung (100) verwendet, die den ersten Sensor (110; 110₁) und den zweiten Sensor (120) aufweist, wobei das Verfahren (400) bei einem Initialisieren (140) des Blutdrucküberwachungsintervalls (142) ein Positionieren der Blutdrucküberwachungsvorrichtung (100) in einer vorbestimmten Position (450) aufweist.

17. Ein Computerprogramm zum Bestimmen der Blutdruckwerte bei dem Verfahren gemäß einem der Ansprüche 15 und 16.

## Revendications

1. Dispositif (100) de contrôle de la pression sanguine, comprenant :
un premier capteur (110 ; 110₁) configuré pour obtenir une première donnée (112 ; 112₁) de mesure en mesurant occlusivement (114a) oscillométriquement des valeurs de la pression sanguine,
un deuxième capteur (120) configuré pour obtenir une deuxième donnée (122) de mesure en mesurant (124) non occlusivement des changements de volume du sang,
un troisième capteur (110₂) configuré pour obtenir une troisième donnée (112₂) de mesure en mesurant (114) occlusivement (114a) oscillométriquement des valeurs de la pression sanguine,
une unité (130) de commande configurée pour initialiser (140) et conduire un intervalle (142) de contrôle de la pression sanguine, dans lequel l'unité (130) de commande est configurée
- pour obtenir la première donnée (112 ; 112₁) de mesure du premier capteur (110 ; 110₁) et pour obtenir la troisième donnée (112₂) de mesure du troisième capteur (110₂) et pour déterminer (132) une donnée (134) de référence pour le deuxième capteur (120) sur la base de la première donnée (112 ; 112₁) de mesure et de la troisième donnée (112₂) de mesure lors de l'initialisation (140) de l'intervalle (142) de contrôle de la pression sanguine,
- pour obtenir la deuxième donnée (122) de mesure du deuxième capteur (120) pendant l'intervalle (142) de contrôle de la pression sanguine, et
- pour déterminer (136) des valeurs (138) de la pression sanguine pendant l'intervalle (142) de contrôle de la pression sanguine sur la base de la deuxième donnée (122) de mesure et de la donnée (134) de référence,
un premier coussin (170 ; 170₁) gonflable comprenant le premier capteur (110 ; 110₁), et
un deuxième coussin (170₂) gonflable comprenant le troisième capteur (110₂),
dans lequel le deuxième capteur (120) est en position latéralement entre le premier coussin (170 ; 170₁) gonflable et le deuxième coussin (170₂) gonflable.

2. Dispositif (100) de contrôle de la pression sanguine suivant la revendication 1, dans lequel l'unité (130) de commande est configurée pour mettre à jour la donnée (134) de référence lors de l'initialisation (140) d'un nouvel intervalle (142) de contrôle de la pression sanguine en obtenant une nouvelle première donnée (112 ; 112₁) de mesure du premier capteur (110 ; 110₁) et en déterminant la donnée (134) de référence pour le deuxième capteur (120) sur la base de la nouvelle première donnée (112 ; 112₁) de mesure.

3. Dispositif (100) de contrôle de la pression sanguine suivant la revendication 2, dans lequel l'unité (130) de commande est configurée pour déclencher périodiquement l'initialisation (140) du nouvel intervalle (142) de contrôle de la pression sanguine.

4. Dispositif (100) de contrôle de la pression sanguine suivant la revendication 2, dans lequel l'unité (130) de commande est configurée pour obtenir une deuxième donnée (150) de mesure représentative et
initialiser (140) le nouvel intervalle (142) de contrôle de la pression sanguine dans le cas d'un écart (160), entre une deuxième donnée (122) de mesure en cours obtenue à partir du deuxième capteur (120) pendant l'intervalle (142) de contrôle de la pression sanguine et la deuxième donnée (150) de mesure représentative excédant un seuil (162) déterminé à l'avance.

5. Dispositif (100) de contrôle de la pression sanguine suivant l'une des revendications 1 à 4, dans lequel l'unité (130) de commande est configurée pour conduire la mesure (114) occlusivement (114a) oscillométriquement de valeurs de la pression de la sanguine en
gonflant (172) le premier coussin (170 ; 170₁) gonflable jusqu'à une pression (174) déterminée à l'avance et
vidant (176) peu à peu le premier coussin (170 ; 170₁) gonflable et donnant instruction au premier capteur (110 ; 110₁) de mesurer simultanément des oscillations de la pression dans le coussin pour obtenir la première donnée (112 ; 112₁) de mesure.

6. Dispositif (100) de contrôle de la pression sanguine suivant la revendication 5, dans lequel l'unité (130) de commande est configurée pour obtenir la première donnée (112 ; 112₁) de mesure du premier capteur (110 ; 110₁) et pour déterminer (132) la donnée (134) de référence pour le deuxième capteur (120) sur la base d'au moins deux points (180, 184) de mesure des oscillations mesurées de la pression dans le coussin.

7. Dispositif (100) de contrôle de la pression sanguine suivant l'une des revendications 1 à 6, dans lequel l'unité (130) de commande est configurée pour conduire la mesure (114) occlusivement (114a) oscillométriquement de valeurs de la pression sanguine en
gonflant (172) simultanément le premier coussin (170 ; 170₁) gonflable et le deuxième coussin (170₂) gonflable jusqu'à une pression (174) déterminée à l'avance, et vidant (176) simultanément peu à peu le premier coussin (170 ; 170₁) gonflable et le deuxième coussin (170₂) gonflable et donnant instruction au premier capteur (110 ; 110₁) et au troisième capteur (110₂) de mesurer simultanément des oscillations de la pression dans le coussin pour obtenir la première donnée (112 ; 112₁) de mesure et la troisième donnée (112₂) de mesure.

8. Dispositif (100) de contrôle de la pression sanguine suivant la revendication 7, dans lequel l'unité (130) de commande est configurée pour obtenir la première donnée (112 ; 112₁) de mesure du premier capteur (110 ; 110₁) et pour obtenir la troisième donnée (112₂) de mesure du troisième capteur (110₂) et pour déterminer (132) la donnée (134) de référence pour le deuxième capteur (120) sur la base d'au moins deux points (180, 184) de mesure des oscillations mesurées de la pression dans le coussin de la première donnée (112 ; 112₁) de mesure et sur la base d'au moins deux points (180, 184) de mesure des oscillations mesurées de la pression dans le coussin de la troisième donnée (112₂) de mesure lors de l'initialisation (140) de l'intervalle (142) de contrôle de la pression sanguine.

9. Dispositif (100) de contrôle de la pression sanguine suivant l'une des revendications 1 à 8, dans lequel le premier capteur (110 ; 110₁) et/ou le troisième capteur (110₂) est / sont un capteur de la pression au manomètre.

10. Dispositif (100) de contrôle de la pression sanguine suivant l'une des revendications 1 à 9, dans lequel le deuxième capteur (120) est un capteur photopléthysmographique et/ou dans lequel le deuxième capteur (120) est configuré pour mesurer les changements de volume du sang en utilisant un radar ou des ultrasons et/ou
dans lequel le deuxième capteur (120) est configuré pour mesurer des changements du volume du sang en mesurant un électrocardiogramme.

11. Dispositif (100) de contrôle de la pression sanguine suivant l'une des revendications 1 à 10, configuré pour travailler dans un mode (192) dormant, dans lequel l'unité (130) de commande est configurée pour déterminer, dans le mode (192) dormant, les valeurs de la pression sanguine pendant l'intervalle (142) de contrôle de la pression sanguine sur la base de la deuxième donnée (122) de mesure et de la donnée (134) de référence en utilisant un moyen (200) de traitement pour corriger un biais de dormant.

12. Dispositif (100) de contrôle de la pression sanguine suivant l'une des revendications 1 à 11, configuré pour travailler dans un mode (194) MAP, dans lequel l'unité (130) de commande est configurée pour obtenir, dans le mode (194) MAP, la première donnée (112 ; 112₁) de mesure du premier capteur (110 ; 110₁) pendant l'intervalle (142) de contrôle de la pression sanguine et pour déterminer (136) des valeurs (138) de la pression sanguine pendant l'intervalle (142) de contrôle de la pression sanguine sur la base de la première donnée (112 ; 112₁) de mesure.

13. Dispositif (100) de contrôle de la pression sanguine suivant la revendication 12, dans lequel l'unité (130) est configurée pour déterminer, dans le mode (194) MAP, des valeurs de la pression sanguine systolique et diastolique sur la base de la première donnée (112 ; 112₁) de mesure.

14. Dispositif (100) de contrôle de la pression sanguine suivant l'une des revendications 1 à 13, pour un contrôle non invasif de la pression sanguine à une extrémité d'une personne.

15. Procédé (400) de contrôle de la pression sanguine, comprenant :
mesurer (410) occlusivement (114a) oscillométriquement des valeurs de la pression sanguine en utilisant un premier capteur (110 ; 110₁) pour obtenir une première donnée (112 ; 112₁) de mesure,
mesurer (430 ; 124) non occlusivement des changements de volume du sang en utilisant un deuxième capteur (120) pour obtenir une deuxième donnée (122) de mesure,
mesurer (114) occlusivement (114a) oscillométriquement des valeurs de la pression sanguine en utilisant un troisième capteur (110₂) pour obtenir une troisième donnée (112₂) de mesure,
initialiser (140) et conduire un intervalle (142) de contrôle de la pression sanguine en
- obtenant la première donnée (112 ; 112₁) de mesure du premier capteur (110 ; 110₁) et obtenant la troisième donnée (112₂) de mesure du troisième capteur (110₂) et déterminant (420 ; 132) une donnée (134) de référence pour le deuxième capteur (120) sur la base de la première donnée (112 ; 112₁) de mesure et de la troisième donnée (112₂) de mesure lors de l'initialisation (140) de l'intervalle (142) de contrôle de la pression sanguine,
- obtenant la deuxième donnée (122) de mesure du deuxième capteur (120) pendant l'intervalle (142) de contrôle de la pression sanguine et
- déterminant (440 ; 136) des valeurs de la pression sanguine pendant l'intervalle (142) de contrôle de la pression sanguine sur la base de la deuxième donnée (122) de mesure et de la donnée (134) de référence,
dans lequel le premier capteur (110 ; 110₁) est renfermé par un premier coussin (170 ; 170₁) gonflable,
dans lequel le troisième capteur (110₂) est renfermé par un deuxième coussin (170₂) gonflable, et
dans lequel le deuxième capteur (120) est en position latéralement entre le premier coussin (170 ; 170₁) gonflable et le deuxième coussin (170₂) gonflable.

16. Procédé (400) suivant la revendication 15, utilisant un dispositif (100) de contrôle de la pression sanguine comprenant le premier capteur (110 ; 110₁) et le deuxième capteur (120), dans lequel le procédé (400) comprend mettre le dispositif (100) de contrôle de la pression sanguine dans une position (450) déterminée à l'avance lors d'une initialisation (140) de l'intervalle (142) de contrôle de la pression sanguine.

17. Programme d'ordinateur pour déterminer les valeurs de la pression sanguine dans le procédé suivant l'une des revendications 15 et 16.
